# EUROPÄISCHE PATENTSCHRIFT

(11) **EP 0 612 772 B1**
(45) Veröffentlichungstag und Bekanntmachung des Hinweises auf die Patenterteilung: **04.06.1997**
(21) Anmeldenummer: 94102220.4
(22) Anmeldetag: 14.02.1994
(51) Int. Cl.: C08F 246/00, C08F 30/04, C09K 11/06, G01N 33/533

(54) **Lumineszierende Copolymere**
Luminiscent copolymers
Copolymères luminescents

(30) Priorität: 26.02.1993 DE 4305959
(43) Veröffentlichungstag der Anmeldung: 31.08.1994
(73) Patentinhaber: BAYER AG, 51368 Leverkusen (DE)
(72) Erfinder: Heiliger, Ludger, Dr., D-51373 Leverkusen (DE)

(56) Entgegenhaltungen:
- WO-A-89/08682
- GB-A- 959 679

## Beschreibung

Die Erfindung betrifft lumineszierende Copolymere, Komplexsalze von seltenen Erdmetallen (als Vorprodukte), Verfahren zu deren Herstellung und ihre Verwendung in der medizinischen Diagnostik und zur Markierung von Kunststoffen. Die Copolymere sind aufgebaut aus speziellen lumineszierenden Komplexsalzen von Seltenen Erdmetallen als Monomere und beliebigen Comonomeren. Unter Lumineszenz versteht man die Aussendung elektromagnetischer Strahlung (Licht) bei der Rückkehr von Atomen aus einem angeregten Zustand in den Grundzustand. Dieser Übergang kann aus einem angeregten Singulettzustand oder einem angeregten Triplettzustand erfolgen. Die aus einem Singulettzustand emittierte Strahlung wird als Fluoreszenz bezeichnet, die aus einem Triplettzustand emittierte Strahlung als Phosphoreszenz. Letztere erfordert eine Spinumkehr zwischen Anregung und Energieabgabe, die eine verlängerte Lebensdauer des angeregten Zustandes bewirkt, weshalb die Phosphoreszenz erst nach einer zeitlichen Verzögerung eintritt.

Polymere mit organischen fluoreszierenden Gruppen sind bekannt. In der medizinischen Diagnostik werden diese als Markierungssubstanzen für biologisch aktive Moleküle wie beispielsweise Proteine oder Nukleinsäuren verwendet (z.B. EP-A-513 560, US-PS 41 166 105). Alle organischen fluoreszierenden Verbindungen konkurieren mit der Hintergrund-, d.h. Eigenfluoreszenz des biologischen Materials (Proteine, Nukleinsäuren). Dieses besitzt Abklingzeiten seiner angeregten Zustände in der gleichen Größenordnung wie die Markierungssubstanzen selbst. Die dadurch erhöhten Blindwerte bei der Messung der Fluoreszenz der Markierungssubstanzen vermindern die Nachweisempfindlichkeit beträchtlich unter die theoretisch möglicher Grenze. Durch die längere Lebensdauer des Triplettzustandes sowie die Übertragung von dessen Energie auf das Seltenerdmetallion wird die Fluoreszenz des Metallkation verzögert; sie kann noch gemessen werden, wenn die Emission der biologischen Materialien (Hintergrundfluoreszenz) bereits abgeklungen ist. Das höhere Signal/Rausch-Verhältnis erlaubt daher prinzipiell einen empfindlichen Nachweis des biologischen Materials und daher eine frühere Erkennung von potentiellen Krankheitserregern.

Diagnostische Nachweismethoden mit Seltenerdmetallen sind bekannt (CRC Crit. Rev. Anal. Chem. 1987, 18, 105-154; Scand. J. Clin. Lab. Invest. 1988, 48, 389-400). Dabei werden Eu³⁺ Metallkationen mit Aminopolycarbonsäuren komplexiert und an Proteine (ca. 5 bis 15 Eu³⁺/Protein), beispielsweise Streptavidin oder Antikörper gebunden. Nachdem die Proteine die immunologische Erkennungsreaktionen mit ihren zugehörigen Haptenen, bzw. mit den hapten - gelabelten Gensonden - DNA -Hybriden ausgeführt haben, wird der eigentliche Nachweis durch Lumineszenz erbracht. Dazu muß der nicht lumineszente Eu³⁺-Aminopolycarbonsäurekomplex zerstört und das Eu³⁺ mit neu zugefügten UV-Energieüberträger-Liganden komplexiert werden, was einen zusätzlichen Aufwand mit sich bringt (Waschprozesse, unspezifische Wechselwirkungen). Durch die Notwendigkeit des Umkomplexierens ist die Nachweismethode nur begrenzt anwendbar. Die erfindungsgemäßen lumineszierenden Copolymeren benötigen keine Umkomplexierung, da sie unter den Anwendungsbedingungen stabil sind. Dadurch vereinfacht sich die Handhabung und Durchführung der Nachweisreaktionen.

Neuere Entwicklungen (Anal. Chem. 1990, 62, 1841 - 1845; Clin. Chem. 1990, 36, 1497 - 1502) beschreiben Eu³⁺-Komplexe, die zum Nachweis von Proteinen nicht mehr umkomplexiert werden müssen. Bei diesen ist jedes Eu³⁺-Ion aber nur mit einem UV-Energieüberträgermolekül komplexiert. Um hohe Lumineszenzintensitäten zu erhalten, was einen empfindlichen Nachweis erst ermöglicht, sind aber mehrere UV-Absorber-Liganden pro Metall-Ion nötig; maximal sind vier möglich (J. Inorg. Nucl. Chem. 1966, 28, 3005 -3018). Daher kann bei dieser Methode hohe Nachweisempfindlichkeit nur mit Vielfachmarkierung des Proteins (bis zu 450 Eu³⁺/Protein) erreicht werden, was nur in Ausnahmefällen gelingt. Die erfindungsgemäßen Copolymere haben hohe Lumineszenzintensität, da sie bis zu vier UV-Überträgerliganden pro Metallion besitzen. Die erfindungsgemäßen Copolymere erreichen ferner ein wesentlich höheres Eu³⁺/Protein-Verhältnis (typischerweise zwischen 10³-10⁵), als die bisher beschriebenen. Da es sich bei den erfindunasgemäßen Copolymeren nicht um metallionenmarkierte Proteine (oder andere Biomaterialien) handelt, sondern um synthetische metallionenhaltige Polymerisate, sind sie auch unter Bedingungen einsetzbar, bei denen üblicherweise Proteine denaturiert werden (hohe Temperatur, hohe Ionenstärke der Lösung).

Seltenerdmetallhaltige Polymere sind bekannt und werden beispielsweie in J. Appl. Pol. Sc. 1980, 25, 2007-2017 beschrieben. Dort wurden UV-Energieüberträger als Komplexliganden für Eu³⁺ in Polymere eingebaut und diese mit Eu³⁺ beladen. Es wurde gezeigt, daß die mehrfache Koordination eines Metallions mit den polymeren Liganden durch die wachsende sterische Hinderung und abnehmende freie Beweglichkeit der Einfachbindungen stark eingeschränkt ist, und speziell der stark lumineszierende tetrakoordinierte Komplex überhaupt nicht gebildet wird.

Die EP-A 404 941 beansprucht eine polymerisierbare Mischung zur Herstellung von Lumineszenz und eine Strahlung selektiv absorbierenden Stoffen auf der Basis von flüssigen Monomeren, welche ein Seltenerdsalz einer Carbonsäure enthält, genau genommen, ein Salz einer halogenierten niederen aliphatischen Carbonsäure mit einem seltenen Erdmetall. Das hauptsächlich verwendete Monomer ist ein Alkylmethacrylat, es ist aber auch Styrol genannt. Die Salze der seltenen Erdmetalle sind hauptsächlich solche der Trifluoressigsäure. Diese Salze werden nicht einpolymerisiert, sondern nur physikalisch in dem Polymerisat eingeschlossen. Es handelt sich bei den seltenen Erdmetallverbindungen nicht um Komplexsalze . Die erhaltenen Produkte und die Produkte der vorliegenden Erfindung sind also völlig verschieden. Auch ihre Wirkung ist verschieden.

GB 959 679 lehrt blau fluoreszierende Copolymere eines fluoreszierenen Monomeren mit andern Monomeren. Es werden aber keine Komplexsalze benutzt.

Gegenstand der Erfindung sind lumineszierende Copolymere der Struktur [A]ₐ-[B]_{b},
worin
- A: ein Komplexsalz eines seltenen Erdmetalls der Formel M³⁺ (L)ₙ in polymerisierter Form darstellt, in welchem
M für Dy, Sm, Eu oder Tb steht und
L für einen zweizähnigen Liganden, der ein konjugiertes π-System, mindestens ein Sauerstoff- oder Stickstoffatom und mindestens eine polymerisierbare Doppelbindung enthält und
n für eine ganze Zahl von 1 bis 4, und
- B: ein (Co)monomer in polymerisierter Form bedeutet und
- a und b: die Massenanteile von A und B im Copolymer mit a 0,0001 bis 20 Gew.-% und b 99,9999 bis 80 Gew.-% darstellt.

Die erfindungsgemäßen Copolymere enthalten Seltenerdmetallkationen tetrakoordiniert mit UV-Energieüberträger Liganden; Die Kationen werden vor der Polymerisation komplexiert und der fertige Komplex polymerisiert. Daher sind die erfindungsgemäßen Copolymere an Lumineszenzintensität den in der Literatur beschriebenen weit überlegen.

Die erfindungsgemäßen Copolymere sind ferner geeignet, Kunststoffe zu markieren und sie dadurch unterscheidbar zu machen.

Die zweizähnigen Liganden des Komplexsalzes M³⁺(L)ₙ schließen auch ringförmige und insbesondere aromatische Verbindungen ein. Die Liganden müssen so auf das Zentralmetallion abgestimmt sein, daß eine Energieübertragung möglich ist, d.h. die Emissionswellenlänge der Phosphoreszenz der Liganden darf nur maximal 50 nm kleiner sein, als die Absorptionswellenlänge des Metallions.

n ist eine ganze Zahl von 1 bis 4, bevorzugt 3 oder 4, insbesondere 4.

Als Liganden sind folgende bevorzugt:

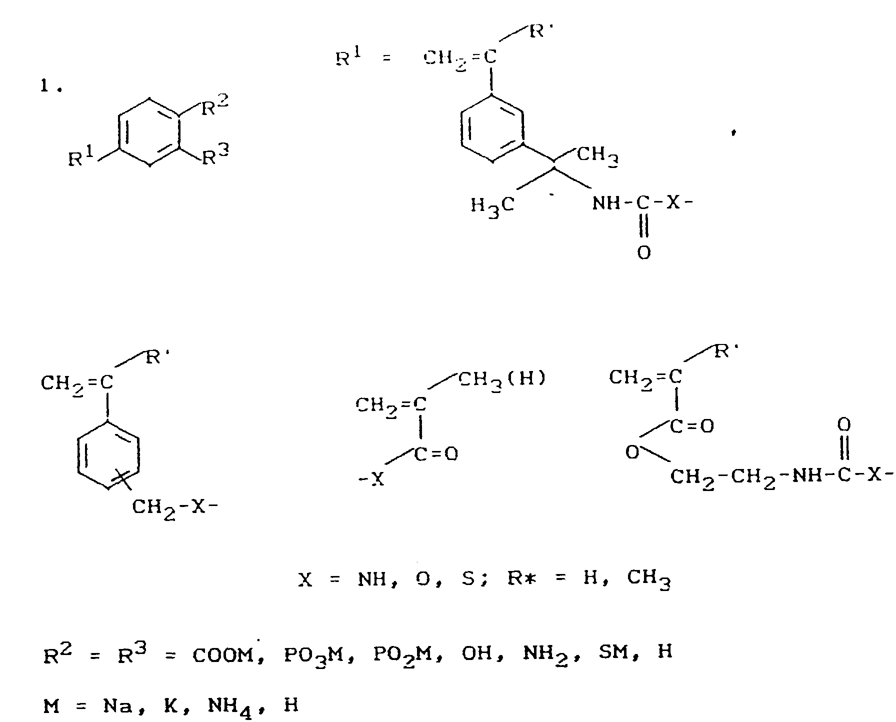

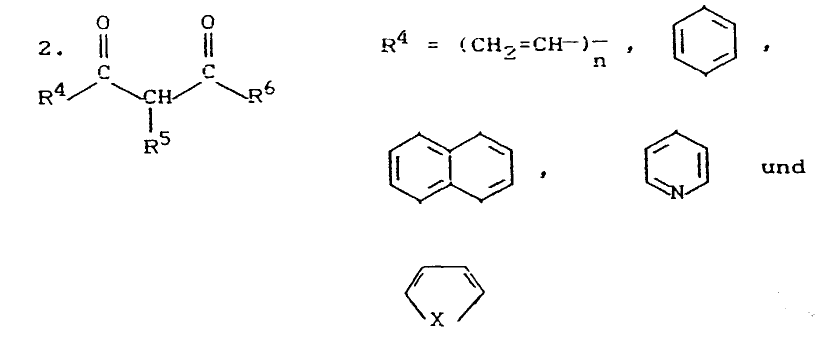

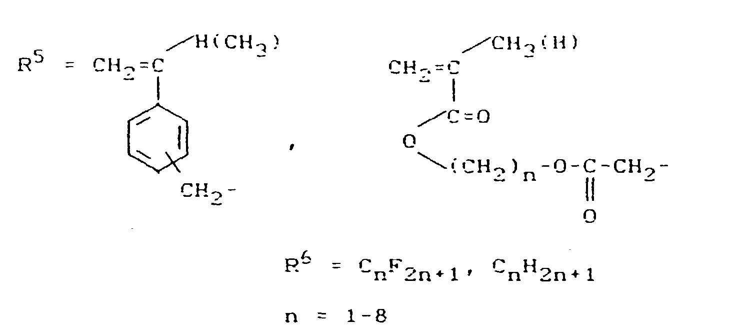

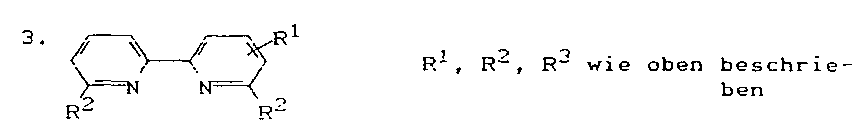

Besonders bevorzugte lumineszierende Komponenten sind:

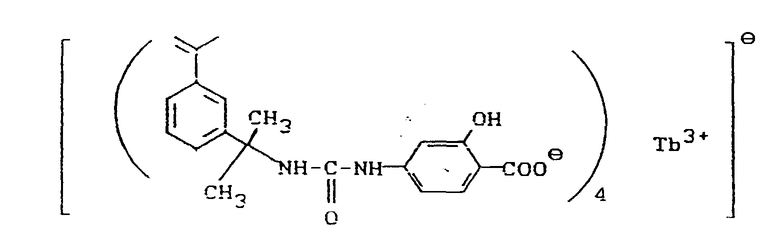

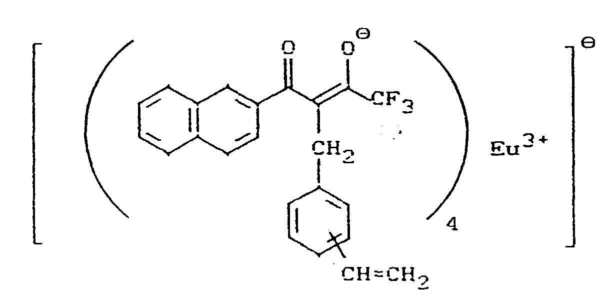

Die polymerisierbaren Liganden werden durch eine Reaktion von geeigneten, funktionelle Gruppen tragenden Monomeren wie beispielsweise (Meth)-Acrylsäurechlorid, Isocyanatoethylmethacrylat, Isopropenyl-α,α-dimethylbenzylisocyanat, Chlormethylstyrol, Chloroacetoxypropyl- oder -ethylmethacrylat mit geeigneten UV Energieüberträgern über Alkyl-, Ester-, Säureamid, Urethan-, Harnstoff- und/oder Thioharnstoff-Gruppierungen gebildet.

Als (Co-)Monomere B kommen in Frage:
α,β-ungesättigte Verbindungen wie Styrol, α-Methylstyrol, Vinyltoluol, substituierte Vinyltoluole, Vinylpyridin, Acrylnitril und die Ester der Acryl- und Methacrylsäure. Bevorzugt sind die Ester der (Meth)Acrylsäure mit 1 bis 20 C-Atomen im Alkoholteil; beispielhaft seien genannt:
   Methyl(meth)acrylat, Ethyl(meth)acrylat, n-Propyl(meth)-acrylat, iso-Propyl(meth)acrylat, n-Butyl(meth)acrylat, iso-Butyl(meth)acrylat, tert.-Butyl(meth)acrylat und 2-Ethylhexyl(meth)acrylat, Gut geeignet sind auch Methacrylsäureester cycloaliphatischer Alkohole, wie Cyclohexylmethacrylat, Furfurylmethacrylat, Vinylester, wie Vinylacetat, -propionat, Maleinsäureanhydrid, Itaconsäureanhydrid,
wobei die Monomeren B auch miteinander in verschiedenen Verhaltnissen gemischt werden können.

Der Massenanteil von a in den erfindungsgemäßen Copolymeren ist 0,0001 bis 20 Gew.-%, bevorzugt 0,01 bis 10 Gew.-%, besonders bevorzugt 0,1 bis 5 Gew.-%.

Der Massenanteil von b ist 99,9999 bis 80 Gew.-%, bevorzugt 99,99 bis 90 Gew.-%, besonders bevorzugt 99,9 bis 95 Gew.-%.

Die erfindungsgemäßen Copolymere können unvernetzt (linear) sein, mit Molmassen von 1.000 bis 10.000.000,
vorzugsweise 5.000 bis 1.000.000, vorzugsweise sind sie jedoch vernetzt. Die erfindungsgemäßen Copolymere können als wäßrige Dispersion vorliegen, mit Polymerteilchen von 30 bis 5.000 nm, vorzugsweise 40 bis 2.000 nm, besonders bevorzugt 50 bis 2.000 nm Durchmesser.

Für diagnostische Anwendungen sind als Comonomere B der erfindungsgemäßen Copolymere polare Monomere wie (Methyl-)Methacrylat, Ethylenglykoldimethacrylat, gegebenenfalls zusammen mit wasserlöslichen Monomeren wie Acrylnitril, (Meth-)Acrylsäure, Hydroxyethyl(meth-)acrylat, Dimethylaminoethylmethacrylat, besonders bevorzugt, die die unspezifischen Wechselwirkungen mit Biomaterialien unterdrücken können. Bevorzugt werden erfindungsgemäße Copolymere in Form von wäßrigen Dispersionen, mit Polymerteilchen von 30 bis 6.000 nm, vorzugsweise 40 bis 3.000 nm, besonders bevorzugt 40 bis 2.000 nm Durchmesser. Es können Metall³⁺/Protein-Verhältnisse von 10² bis 10⁷ erreicht werden, je nach Partikeldurchmesser.

Die wäßrigen Dispersionen können nach bekannten Verfahren der Emulsions-, bzw. Suspensionspolymerisation hergestellt werden, wobei als Radikalinitiatoren beispielsweise Azobisisobutyronitril, Benzoylperoxid oder Alkali, bzw. Ammonium Peroxydisulfat verwendet werden können. Dazu werden die meist wasserunlöslichen lumineszierenden Komponenten A und die Comonomeren B mit Hilfe eines Emulgators bzw. Dispergators in Wasser emulgiert, bzw. dispergiert und dann polymerisiert. Als Emulgator bzw. Dispergator eignen sich beispielsweise Alkali- oder Ammonium-Salze von Alkylsulfaten sowie Alkylsulfonaten (C₈-C₂₂), Alkylbenzolsulfonaten (C₆-C₁₅) oder Fettsäuren. Ferner können polymere Emulgatoren bzw. Dispergatoren wie beispielsweise Polyvinylpyrolidon, Polyvinylalkohol oder verseifte und teilverseifte Styrol/ Maleinanhydrid-Copolymerisats oder die in EP-A-334 032 beschriebenen Polyesterurethane verwendet werden. Als Saat-Latex eignen sich beispielsweise Polystyrol- oder Polymethylmethacrylat-Latizes bzw. Dispersionen. Der Latex bzw. die Dispersion kann von eventuell verbliebenen, molekular gelösten Bestandteilen durch Zentrifugation gereinigt werden. Dieses Produkt kann direkt mit biologisch relevanten Molekülen wie beispielsweise Proteinen, Antikörpern, aminierten Gensonden über eine Carbodiimid-Kopplung kovalent verknüpft werden und in diagnostischen Nachweissystemen eingesetzt werden.

Bei der Anwendung der erfindungsgemäßen Copolymeren zur Kunststoffmarkierung richtet sich die Wahl der Monomeren B nach dem Kunststoff. Beispielsweise ist zur Markierung von Polymethylmethacrylat und von Polyamiden wie Polyamid-6 oder Polyamid-6,6, Methylmethacrylat oder Acrylamid als Monomer B besonders bevorzugt. Zur Markierung von Polystyrol, Styrol/Butadien-Copolymerisat und Poly(oxycarbonyloxy-1,4-phenylenisopropyliden-1,4-phenylen) ist Styrol als Monomer B besonders bevorzugt. Zur Markierung von Polyethylen, Polypropylen und deren Copolymeren wie Ethylenvinylacetat-Copolymer ist Stearylmethacrylat gegebenenfalls zusammen mit Methylmethacrylat im Gewichtsverhältnis 10:1 bis 30:1 als Monomer B besonders bevorzugt. Zur Markierung von Polyethylenterephthalat, Styrol/Butadien-Acrylnitrilterpolymerisat und von Styrol/Acrylnitril-Copolymerisat ist eine Mischung aus 72 Gew.-% Styrol und 28 Gew.-% Acrylnitril als Comonomere B besonders bevorzugt.

Es können auch bereits markierte Kunststoffe als Marker für weitere Kunststoffe eingesetzt werden. So läßt sich ein mit einem erfindungsgemäßen Copolymeren aus einer lumineszierenden Komponente A und Methylmethacrylat als Monomer B markiertes Polymethylmethacrylat zur Markierung von Polyamiden verwenden.

Die erfindungsgemäßen Copolymeren für die Kunststoffmarkierung können durch Emulsionspolymerisation hergestellt werden, wobei nicht der Latex selbst, sondern das daraus isolierte Copolymer zur Markierung verwendet Kunststoffen mit den erfindungsgemäßen Copolymeren (oder deren Legierungen) ist durch mechanisches Mischen, Kneten und/oder Extrudieren möglich. Beispielsweise kann der zu markierende Kunststoff mit dem erfindungsgemäßen Copolymer bei einer Temperatur mindestens 10°C oberhalb der Erweichungstemperatur des Bestandteils mit der höchsten Erweichungstemperatur vermischt, verknetet oder extrudiert werden. Üblicherweise beträgt die erforderliche Menge des erfindungsgemäßen Copolymer 0,001 bis 10 Gew.-%, bevorzugt 0,005 bis 5 Gew.-%, besonders bevorzugt 0,01 bis 2 Gew.-% des markierten Kunststoffs.

### Beispiele

### Lumineszierende Komponenten

### Beispiel 1

a) 0,05 Mol (7,65 g) p-Aminosalicylsäure werden mit 0,05 Mol (10,05 g) m-TMI® (α-α-Dimethyl-m-isopropenylbenzylisocyanat) und einer Spatelspitze 2,6-Bis^{t}butylphenol in 100 ml trockenem Aceton 6 Stunden unter Rückfluß erhitzt. Der entstandene Niederschlag wird abgesaugt, mit kaltem Aceton nachgewaschen und getrocknet. Die ¹H-NMR und IR-Analyse ergibt N-(α,α-Dimethyl-m-isopropenylbenzyl)-N'(-p-salicyl)harnstoff als reines Produkt. (δ¹H 6,7 und 8,8 ppm, υ_{C-H} Harnstoff = 1.630 cm⁻¹)
   Ausbeute ca. 63 %
b) NH₄ [Tb(C₂₀H₂₁N₂O₄)₄]
   0,2 g Ligand aus Beispiel la) und 0,06 g Tb(NO₃)₃·5H₂O werden separat in je 3 ml Methanol gelöst. Die beiden Lösungen werden vereinigt und 0,5 ml konzentrierte Ammoniaklösung zugetropft, wobei sich ein Niederschlag bildet. Dieser wird abgesaugt, mit Wasser gewaschen und im Hochvakuum getrocknet. Der ¹⁵⁹Tb-Gehalt wurde elementaranalytisch zu 10,1 % Tb bestimmt.

### Beispiel 2

a) 0,05 Mol (13,25 g) 1-(2-Naphthoyl)-3,3,3-trifluoroaceton werden in 50 ml Methanol gelöst. Dann wird 0,05 Mol (11,32 g) Natriummethanolat aus einer 30 Gew.-%igen Natriummethanolat-Lösung in Methanol langsam bei 0°C zudosiert, auf Zimmertemperatur erwärmt, 0,05 Mol (7,63 g) Chlormethylstyrol zugegeben und 16 Stunden auf Rückfluß erhitzt. Der entstandene feine Niederschlag (NaCl) wird abfiltriert, die Lösung im Wasserstrahlvakuum auf ca. 1/3 des Ausgangsvolumens eingeengt und mit soviel Wasser versetzt, daß sich der entstandene Niederschlag gerade löst. Die organische Phase wird abgetrennt, die wäßrige Phase zweimal mit Chloroform ausgeschüttelt, die vereinigten organischen Phasen über Natriumsulfat getrocknet, filtriert und eingedampft. Die Rohprodukte werden auf einer Kieselgelsäule (0,063 bis 0,2 mm) in einem Lösungsmittelgemisch aus Toluol:Methylenchlorid:Ethylacetat: Methanol im Verhältnis 5:3:1:0,5 gereinigt. (δ¹⁹F-76,6 ppm, Ausbeute an 1-(2-Naphthoyl)-1-chlormethylstyrol-3,3,3-trifluoroaceton: ca. 25 % der Theorie.
b) NH₄[Eu(C₂₃H₁₆F₃O₂)₄]
   0,1 g Ligand aus Beispiel 2a) und 0,023 g EuCl₃·6 H₂O werden separat in 2 ml Methanol gelöst. Die beiden Lösungen werden vereinigt und 0,2 ml konzentrierte Ammoniaklösung zugetropft, wobei sich ein Niederschlag bildet. Dieser wird abgesaugt, mit Wasser gewaschen und im Hochvakuum getrocknet. Der ¹⁵²Eu-Gehalt wurde elementaranalytisch zu 9,0 % En bestimmt.

### Copolymere als Marker für Polyethylen (PE)

### Beispiel 3

0,25 g EuCl₃·6H₂O, 1,575 g Substanz aus Beispiel 2a) und 0,645 g N,N-Dimethylaminoethylmethacrylat werden in 135,5 ml Methanol gelöst und die Komplexbildung durch UV-Anregung bei 366 nm geprüft (starkes rotes Leuchten). Dann wird 3,57 g Ethylenglykoldimethacrylat, 28,5 g Stearylmethacrylat und 0,35 g Azobisisobutyronitril zugegeben, die Apparatur evakuiert, mit Reinststickstoff belüftet, dieser Vorgang noch zweimal wiederholt und auf 65°C erhitzt. Bereits nach wenigen Stunden zeigt sich ein weißer Niederschlag, wobei nach 16 h Reaktionszeit der gesamte Ansatz vollständig ausgefallen ist. Der Niederschlag wird abgesaugt, mit Methanol nachgewaschen und getrocknet. Ausbeute: 90 % der Theorie eines unter UV-Anregung intensiv rot leuchtenden Pulvers.

### Beispiel 4

Analog Beispiel 3 werden 0,25 g Tb(NO₃)₃·5H₂O mit 1,23 g Substanz aus Beispiel la) und 0,55 g N,N'-Dimethylaminoethylmethacrylat in 54,9 ml Dimethylacetamid komplexiert (grünes Leuchten nach Anregung bei 366 nm) und nach Zugabe von 1,72 g Ethylenglykoldimethacrylat, 13,7 g Stearylmethacrylat und 0,17 g Azobisisobutyronitril 16 h bei 65°C polymerisiert. Nach Absaugen des Niederschlages und Waschen mit Methanol wird das Filtrat in Methanol gefällt, abgesaugt, nachgewaschen und getrocknet. Ausbeute: 89 % der Theorie eines unter UV-Anregung intensiv grün leuchtenden Pulvers.

### Copolymere als Marker für Polymethylmethacrylat

### Beispiel 5 und 6

Die Beispiele 3 und 4 werden mit folgender Änderung wiederholt: statt Stearylmethacrylat wird die gleiche Menge Methylmethacrylat verwendet.

### Einarbeitung der Polymermarker in Polyethylen

### Beispiel 7

Ein Haake Rheometer Kneter, Fassungsvolumen 50 ml, wird auf 130°C geheizt und 44 g Polyethylen-Granulat (LDPE, Firma Novex Exp. 2184) eingefüllt. Nach 10 Minuten Kneten werden 0,37 g Substanz aus Beispiel 3 sowie 0,185 g Substanz aus Beispiel 4 zugegeben und noch 15 Minuten bei dieser Temperatur geknetet. Das Polymerisat enthält somit je ca. 20 bis 25 ppm Metallionen und Farbstoffe.

### Einarbeitung der Polymermarker in Polymethylmethacrylat

### Beispiel 8

Der Versuch aus Beispiel 7 wird mit folgenden Änderungen wiederholt:

Polyethylen-Granulat wird durch granuliertes Polymethylmethacrylat (Röhm GmbH, Darmstadt), sowie die Substanzen aus Beispiel 3 und 4 werden durch die Produkte aus Beispiel 5 und 6 ersetzt und die Knetertemperatur bei 200°C gehalten.

Herstellung von Copolymeren für diagnostische Anwendungen

### Beispiel 9

0,25 g EuCl₃·6H₂O, 1,575 g Substanz aus Beispiel 2 und 0,645 g N,N'-Dimethylaminoethylmethacrylat werden in 135,5 ml Methanol gelöst und die Komplexbildung durch UV-Anregung bei 366 nm geprüft (starkes rotes Leuchten). Dann wird das Methanol im Hochvakuum bei Zimmertemperatur abdestilliert und 3,57 g Ethylenglykoldimethacrylat und 28,5 g Methylmethacrylat zugegeben. Diese Lösung wird zu einer wäßrigen Dispersion aus 8 g Polyesterurethan gemäß EP-A-334 032 Beispiel Oligourethan 1, 0,6 g 4,4'-Azobis-(4-cyanopentancarbonsäure) und 700 ml entionisiertem Wasser, gegeben, 30 Minuten gerührt und auf 65°C erhitzt und 16 Stunden gerührt. Die Rohemulsion wird durch ein Polyamidtuch mit Maschenweite 30 µm filtriert und durch dreimaliges Zentrifugieren und Wiederauffüllen mit entionisiertem Wasser von eventuellen monomeren Verunreinigungen gereinigt. Der pH-Wert der Emulsion wird mit Natronlauge auf 8,5 eingestellt. Die Emulsion besitzt Teilchen eines Durchmessers von 140 nm, entsprechend ca. 12.000 Eu³⁺ Metallatome pro Latexteilchen.

Die Nachweisgrenze der Emulsion liegt bei normaler Blitzlampen-Anregung bei 331 nm und Detektion 615 nm bei 10⁻¹¹ mol Eu³⁺ Ionen pro Liter, d.h. bei ca. 10⁻¹⁵ mol Latexteilchen pro Liter. Die Quantenausbeute der Latexlösung liegt bei Einstrahlung von 331 nm bei 15 %. Die Emulsion kann direkt zum Markieren von Proteinen wie beispielsweise Streptavidin, biologisch relevanten Antikörpern oder aminierten Gensonden verwendet werden.

### Beispiel 10

Der Ansatz von Beispiel 9 wird, mit folgender Änderung wiederholt:

Statt 0,25 g EuCl₃·6H₂O und 1,575 g Substanz aus Beispiel 2 werden 0,38 g Tb(NO₃)₃·5H₂O mit 1,45 g Substanz aus Beispiel 1 verwendet. Der resultierende Latex hat einen mittleren Teilchendurchmesser von 120 nm und fluoresziert bei 546 nm unter Anregung bei 333 nm. Die Tb³⁺-Konzentration errechnet sich zu 7.500 pro Latexteilchen.

## Patentansprüche

1. Lumineszierende Copolymere der Struktur [A]ₐ-[B]_{b},
worin
A ein Komplexsalz eines seltenen Erdmetalls der Formel M³⁺ (L)ₙ in polymerisierter Form darstellt, in welchem
M für Dy, Sm, Eu oder Tb steht und
L für einen zweizähnigen Liganden, der ein konjugiertes π-System, mindestens ein Sauerstoff- oder Stickstoffatom und mindestens eine polymerisierbare Doppelbindung enthält und
n für eine ganze Zahl von 1 bis 4, und
B ein (Co)monomer in polymerisierter Form bedeutet und
a und b die Massenanteile von A und B im Copolymer mit a 0,0001 bis 20 Gew.-% und b 99,9999 bis 80 Gew.-% darstellt.

2. Seltene Erdmetallkomplexe der Formeln

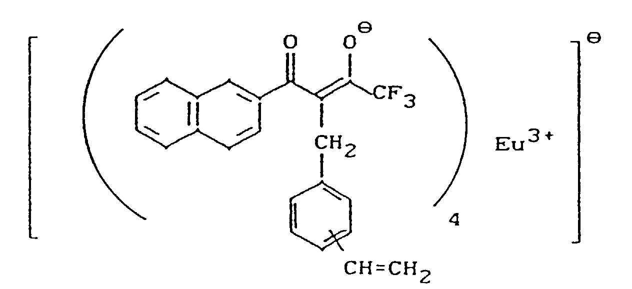

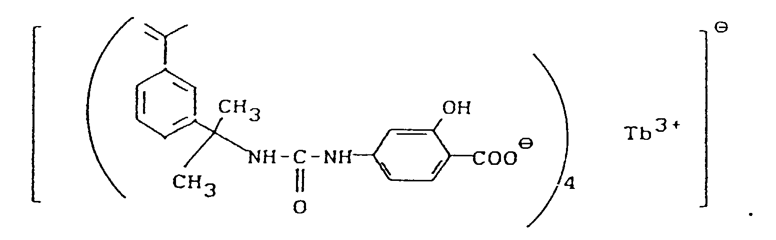

3. Verwendung der lumineszierenden Copolymere gemäß Anspruch 1 in der medizinischen Diagnostik.

## Revendications

1. Copolymères luminescents de structure [A]ₐ-[B]_{b},
dans laquelle
A est un sel complexe, à l'état polymérisé, d'un métal des terres rares, de formule M⁺³ (L)ₙ dans laquelle
M représente Dy, Sm, Eu ou Tb et
L représente un ligand bidentique qui contient un système π conjugué, au moins un atome d'oxygène ou d'azote et au moins une double liaison polymérisable et
n est un nombre entier allant de 1 à 4, et
B est un (co)monomère à l'état polymérisé et a et b sont les proportions en poids de A et B dans le copolymère, a allant de 0,0001 à 20 % en poids et b de 99,9999 à 80 % en poids.

2. Complexes de métaux des terres rares de formules :

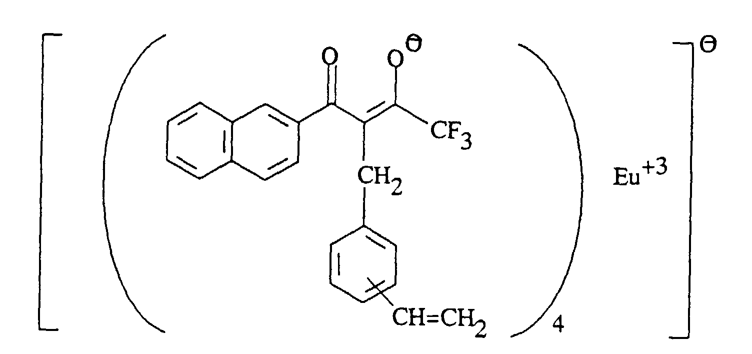

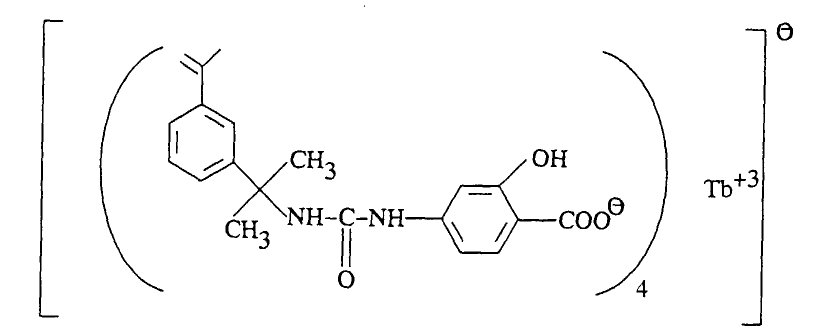

3. Utilisation des copolymères luminescents selon la revendication 1 dans le diagnostic médical.

## Claims

1. Luminescent copolymers of the structure [A]ₐ-[B]_{b},
wherein
A is a complex salt of a rare earth metal of the formula M³⁺(L)ₙ in polymerized form, in which
M is Dy, Sm, Eu or Tb,
L is a bidentate ligand containing a conjugated π system, at least one oxygen or nitrogen atom and at least one polymerizable double bond, and
n is an integer from 1 to 4,
B is a (co)monomer in polymerized form, and
a and b are the proportions of A and B in the copolymer, where a is 0.0001 to 20 wt.% and b is 99.9999 to 80 wt.%.

2. Rare earth metal complexes of the formulae

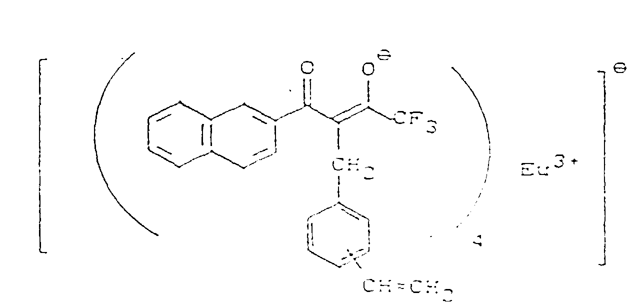

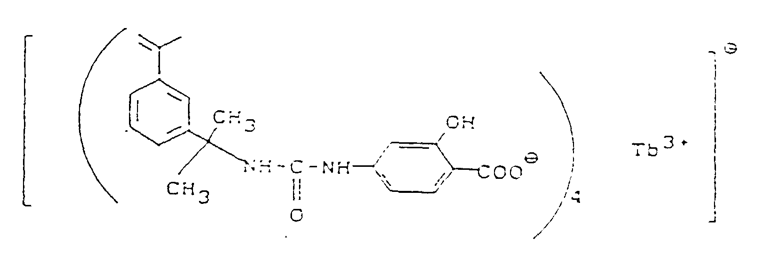

3. Use of the luminescent copolymers according to Claim 1 in medical diagnostics.
